# EUROPEAN PATENT SPECIFICATION

(11) **EP 4 302 810 B1**
(45) Date of publication and mention of the grant of the patent: **25.06.2025**
(21) Application number: 22791782.0
(22) Date of filing: 21.04.2022
(51) Int. Cl.: G16H 20/10, G16H 20/13, G16H 20/40, G16H 20/90, A61B 5/00, A61B 7/00, A61B 7/04, A61B 10/00, A61F 7/00, A61M 11/02, A61M 15/08, A61M 21/00, A61M 21/02, A61N 1/04, A61N 1/36

(54) **INTESTINAL ACTIVITY REGULATION DEVICE, INTESTINAL ACTIVITY REGULATION SYSTEM, AND INTESTINAL ACTIVITY REGULATION PROGRAM**
VORRICHTUNG ZUR REGULIERUNG DER DARMAKTIVITÄT, SYSTEM ZUR REGULIERUNG DER DARMAKTIVITÄT UND PROGRAMM ZUR REGULIERUNG DER DARMAKTIVITÄT
DISPOSITIF DE RÉGULATION DE L'ACTIVITÉ INTESTINALE, SYSTÈME DE RÉGULATION DE L'ACTIVITÉ INTESTINALE ET PROGRAMME DE RÉGULATION DE L'ACTIVITÉ INTESTINALE

(30) Priority: 23.04.2021 JP 2021073438
(43) Date of publication of application: 10.01.2024
(73) Proprietor: Daikin Industries, Ltd., Osaka-shi, Osaka 530-0001 (JP)
(72) Inventor: YAMADA, Nozomi, Osaka-shi, Osaka 530-0001 (JP); MIKI, Sanae, Osaka-shi, Osaka 530-0001 (JP); HIRANO, Tomoya, Osaka-shi, Osaka 530-0001 (JP); HIRAYAMA, Takahiro, Osaka-shi, Osaka 530-0001 (JP); KATO, Masahiro, Osaka-shi, Osaka 530-0001 (JP)
(74) Representative: Goddar, Heinz J.
(86) International application number: PCT/JP2022/018421
(87) International publication number: WO 2022/225011

(56) References cited:
- WO-A1-2020/121786
- CN-U- 212 574 890
- CN-U- 212 574 890
- JP-A- 2003 530 169
- JP-A- 2007 032 088
- JP-A- 2013 007 042
- US-A1- 2014 088 462
- US-A1- 2021 000 999

## Description

### TECHNICAL FIELD

The present invention relates to an intestinal activity regulation device, an intestinal activity regulation system, and an intestinal activity regulation program.

### BACKGROUND ART

Conventionally, a measurement device that measures information on a biological body has been known. Patent Document 1 discloses an intestinal sound measurement device that measures intestinal sound as biological information. The intestinal sound measurement device of Patent Document 1 measures intestinal sound of a target person, and analyzes the measured intestinal sound to grasp the status of peristaltic motion of an intestine.

US 2014/088462 A1 discloses an intestinal activity regulation device for improving intestinal activity of a target person, comprising a regulator that performs a regulation process of regulating the intestinal activity based on a state of the intestinal activity detected by detector that detects that the state of the intestinal activity of the target person.

JP 2013 007042 A teaches that the aroma of a specific essential oil, including lavender oil, promotes the activity of the digestive tract.

US 2021/000999 A1 teaches an aroma diffuser, comprising an acquisition unit that acquires person information on an activity and/or a mood of a person in target space and determines an aroma to be diffused for the identified activity. The aroma diffuser may include a plurality of aroma reservoirs that respectively store different aromas, such as floral-based aroma. The aroma diffuser may also comprise a microphone to acquire a sound which a person in the target space makes.

### CITATION LIST

### PATENT DOCUMENT

Patent Document 1: Japanese Unexamined Patent Publication No. 2016-36637

### SUMMARY OF THE INVENTION

### TECHNICAL PROBLEM

For example, when a person is subjected to mental or physical stress, the activity of the intestine changes due to the stress. When the intestinal activity changes due to, e.g., the stress, symptoms such as constipation and diarrhea may occur. In the intestinal sound measurement device of Patent Document 1, a change in the intestinal activity due to, e.g., influence of the stress can be grasped, but the changed intestinal activity cannot be returned to a normal state.

It is an object of the present invention to regulate the intestinal activity based on the state of the intestinal activity of the target person.

### SOLUTION TO THE PROBLEM

A first aspect of the present invention is directed to an intestinal activity regulation device (2) according to claim 1.

In the first aspect, since the regulator (3) that performs the regulation process of regulating the intestinal activity is provided, the intestinal activity of the target person (T) can be regulated based on the state of the intestinal activity of the target person (T).

A second aspect of the present invention is an embodiment of the first aspect. In the second aspect, the regulator (3) performs the regulation process so as to act on the autonomic nerve of the target person (T).

In the second aspect, the regulator (3) performs the regulation process to act on the autonomic nerve of the target person (T), thereby regulating the intestinal activity of the target person (T).

A third aspect of the present invention is an embodiment of the first or second aspect. In the third aspect, the regulator (3) performs, as the regulation process, a promotion process of promoting the intestinal activity.

In the third aspect, the promotion process as the regulation process is performed, so that the intestinal activity of the target person (T) is promoted.

A fourth aspect of the present invention is an embodiment of any one of the first to third aspects. In the fourth aspect, the regulator (3) performs, as the regulation process, an inhibition process of inhibiting the intestinal activity.

In the fourth aspect, the inhibition process as the regulation process is performed, so that the intestinal activity of the target person (T) is inhibited.

A fifth aspect of the present invention is an embodiment of any one of the first to fourth aspects. In the fifth aspect, the intestinal activity regulation device (2) further includes a display (53) that displays the state of the intestinal activity detected by the detector (11).

In the fifth aspect, the display (53) is provided, so that it is possible to notify the target person (T) of the state of the intestinal activity.

A sixth aspect of the present invention is an embodiment of the fifth aspect. In the sixth aspect, the display (53) displays the state of the intestinal activity before the regulation process and the state of the intestinal activity after the regulation process.

In the sixth aspect, the states of the intestinal activity before and after the regulation process are displayed on the display (53), so that it is possible to notify the target person (T) of a change in the state of the intestinal activity due to the regulation process.

According to the present invention, the regulator (3) has a releaser (20).
, so that the useful substance can be released into the space (S) where the target person (T) is present.

A seventh aspect of the present invention is an embodiment of the first aspect. In the seventh aspect, the regulator (3) causes the releaser (20) to release the useful substance for promoting the intestinal activity when the level of the intestinal activity detected by the detector (11) is a predetermined value or less.

In the seventh aspect, the useful substance is released when the level of the intestinal activity of the target person (T) is the predetermined value or less. With this configuration, when the level of the intestinal activity of the target person (T) is low, the intestinal activity of the target person (T) can be promoted.

An eighth aspect of the present invention is an embodiment of the first or seventh aspect. In the eighth aspect, the regulator (3) further has a blower (30) that blows air in conjunction with the regulation process.

In the eighth aspect, since air is blown by the air blower (30) in conjunction with the regulation process, unevenness in the concentration of the useful substance released from the regulator (3) can be reduced.

A ninth aspect of the present invention is an embodiment of any one of the fist and seventh to eighth aspects. In the tenth aspect, the regulator (3) adjusts the release amount of the useful substance according to environment in the space (S).

In the ninth aspect, the release amount of the useful substance is adjusted according to the environment in the space (S) where the target person (T) is present.

A tenth aspect of the present invention is an embodiment of any one of the first and seventh to ninth aspects. In the tenth aspect, the releaser (20) has a plurality of useful substances of different types, and the regulator (3) adjusts the release amount of the useful substance according to the type of useful substance.

In the tenth aspect, the release amount of the useful substance is adjusted according to the type of useful substance.

An eleventh aspect of the present invention is directed to an intestinal activity regulation system including a detector (11) that detects the state of intestinal activity of a target person (T), and the intestinal activity regulation device (2) of any one of the first to tenth aspects.

### BRIEF DESCRIPTION OF THE DRAWINGS

FIG. 1 is a schematic view of an entire configuration of a space to which an intestinal activity regulation system according to an embodiment is applied.
FIG. 2 is a block diagram of the intestinal activity regulation system.
FIG. 3 is a schematic view of the configuration of a releaser.
FIG. 4 is a table of the types of input information for determining the release amount of a useful substance.
FIG. 5 is a view illustrating the indication of the state of intestinal activity before and after a regulation process on a display.
FIG. 6 is a flowchart of operation of the intestinal activity regulation system.
FIG. 7 is a flowchart of regulation operation.
FIG. 8 is a flowchart of regulation operation according to a first variation.
FIG. 9 is a table of the levels of intensity of aroma according to a second variation.

### DESCRIPTION OF EMBODIMENTS

Embodiments of the present disclosure will be described below with reference to the drawings. The following embodiments are merely exemplary ones in nature, and is not intended to limit the scope, applications, or use of the present invention.

### <<Embodiments>>

### -Outline of Intestinal Activity System-

An intestinal activity regulation system (1) of the present disclosure is a system for improving intestinal activity of a target person (T). The intestinal activity regulation system (1) includes a detection unit (10) and an intestinal activity regulation device (2). The detection unit (10) is a device for detecting the state of the intestinal activity of the target person (T). The intestinal activity regulation device (2) is a device for improving the intestinal activity of the target person (T). Specifically, the intestinal activity regulation device (2) regulates the intestinal activity of the target person (T) based on output from the detection unit (10). The intestinal activity regulation device (2) includes a regulator (3) and a communication terminal (50).

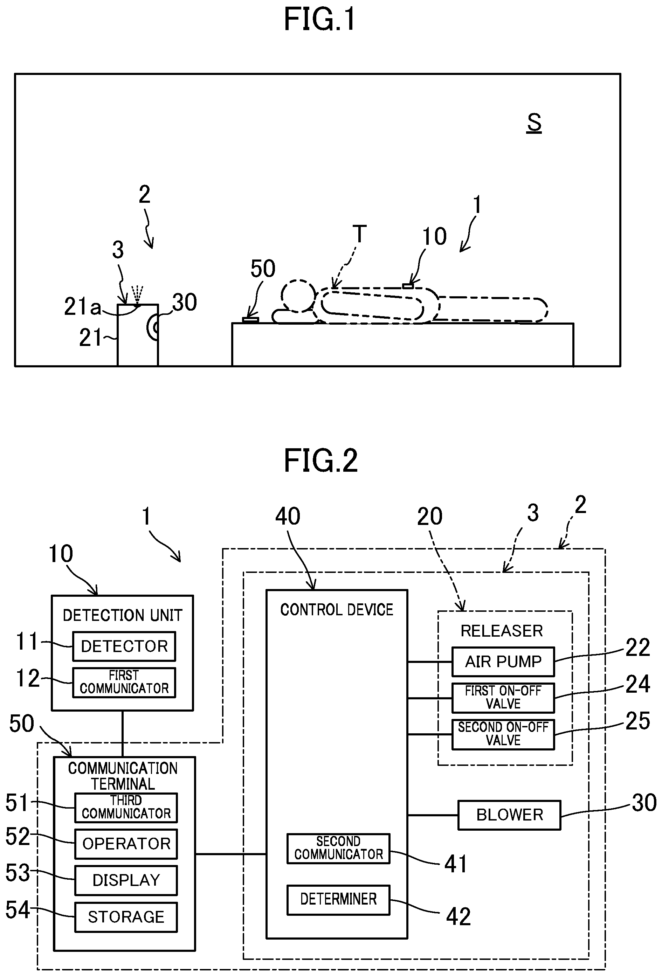

As illustrated in FIG. 1, the intestinal activity regulation system (1) is used in an indoor space (S) where the target person (T) is present. The target person (T) sleeps on bedding such as a bed in the indoor space (S), for example. The detection unit (10) is a small portable device. The detection unit (10) is attached in close contact with the abdomen of the target person (T). The regulator (3) of the intestinal activity regulation device (2) is arranged in the indoor space (S) (for example, on the floor). The communication terminal (50) of the intestinal activity regulation device (2) is a device capable of communicating with the regulator (3) and the detection unit (10). Examples of the communication terminal (50) include a smartphone, a tablet terminal, and a mobile phone.

### <Detection Unit>

As illustrated in FIG. 2, the detection unit (10) includes a detector (11) and a first communicator (12) as functional units. The term "functional unit" described herein or below includes a functional unit implemented only by hardware, a functional unit implemented only by software, and a functional unit implemented by cooperation of hardware and software.

The detector (11) detects the state of the intestinal activity of the target person (T). Here, the intestinal activity is peristaltic motion of an intestine. The peristaltic motion of the intestine is motion in which the intestine repeats contraction and relaxation. By such motion, the contents of the intestine are moved and discharged to the outside of a body. As the intestine undergoes the peristaltic motion, sound is generated due to movement of the contents of the intestine. The detector (11) of this example is a microphone that measures sound (hereinafter referred to as intestinal sound) generated due to the peristaltic motion of the intestine. **In** other words, the detector (11) detects intestinal sound data. The detector (11) may process noise of the measured intestinal sound.

The first communicator (12) is connected to the detector (11) via a wired or wireless communication line. The intestinal sound data detected by the detector (11) is received by the first communicator (12). The first communicator (12) is connected to the communication terminal (50) via short-range wireless communication such as the Bluetooth (registered trademark, the same applies hereinafter). The first communicator (12) transmits the intestinal sound data to the communication terminal (50).

### <Regulator>

As illustrated in FIG. 2, the intestinal activity regulation device (2) includes the regulator (3). The regulator (3) regulates the intestinal activity based on the state of the intestinal activity detected by the detector (11). The regulator (3) includes, as functional units, a releaser (20), a blower (30), and a control device (40).

### <Releaser>

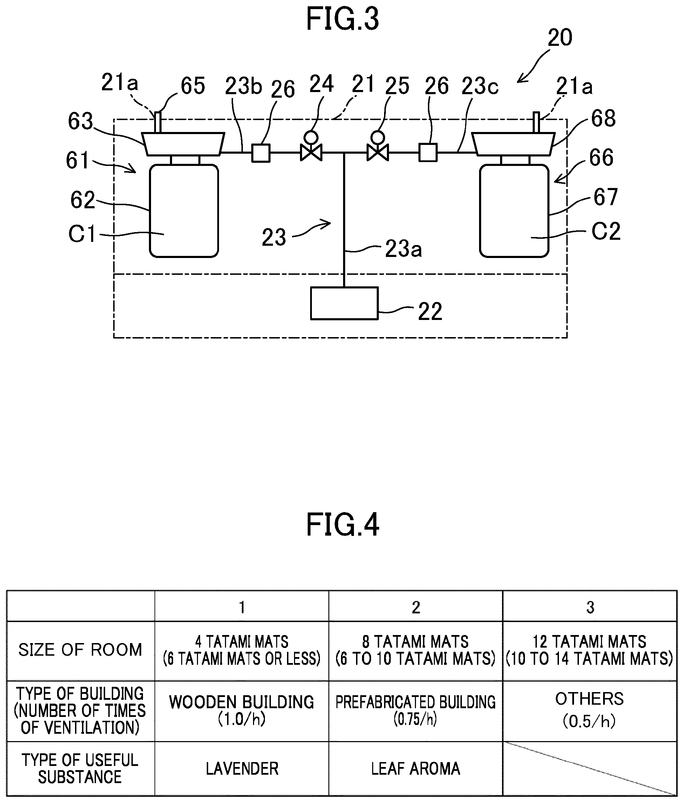

The releaser (20) releases a useful substance into the indoor space (S) where the target person (T) is present. The releaser (20) has a plurality of useful substances of different types. The releaser (20) of this example is a spraying device for releasing the useful substance in the form of mist. The useful substance of this example is a liquid fragrance. In this example, the releaser (20) has two types of fragrance. The number of types of fragrance described here is merely an example. The releaser (20) may have one type or three or more types of fragrance. As illustrated in FIG. 3, the releaser (20) includes a casing (21), spraying units (61, 66), an air pump (22), an air flow path (23), on-off valves (24, 25), and filters (26).

### (Casing)

The casing (21) is formed in a hollow shape. The casing (21) houses the spraying units (61, 66), the air pump (22), the air flow path (23), the on-off valves (24, 25), and the filters (26). A supply port (21a) for supplying the atomized useful substance into the indoor space (S) is formed in an upper portion of the casing (21).

### (Spraying Unit)

The casing (21) houses the two spraying units (61, 66). The first spraying unit (61) has a first tank (62) and a first spraying nozzle (63). The first tank (62) is detachably attached to a lower portion of the first spraying nozzle (63). The first tank (62) stores a first fragrance (C1).

The first spraying nozzle (63) forms an atomizing mechanism for atomizing the first fragrance (C1) in the first tank (62). Specifically, the first spraying nozzle (63) atomizes a liquid film of the first fragrance (C1) using shearing force of an airflow. An ejection port (65) for ejecting the atomized useful substance is formed in an upper portion of the first spraying nozzle (63). The ejection port (65) is exposed from the supply port (21a) of the casing (21) to the indoor space (S).

The configuration of the second spraying unit (66) is similar to the configuration of the first spraying unit (61). A second tank (67) of the second spraying unit (66) stores a second fragrance (C2).

### (Air Pump)

The air pump (22) sucks air in the indoor space (S), and discharges the sucked air to the air flow path (23). The air pump (22) supplies air to the first spraying nozzle (63) and a second spraying nozzle (68).

### (Air Flow Path)

The air pump (22) has a discharge portion connected to the air flow path (23). The air flow path (23) is formed by a tube. The air flow path (23) includes a main flow path (23a), a first branch path (23b), and a second branch path (23c).

The main flow path (23a) has an inflow end connected to the air pump (22). The main flow path (23a) has an outflow end connected to the inflow end of the first branch path (23b) and the inflow end of the second branch path (23c). In other words, the main flow path (23a) branches into the first branch path (23b) and the second branch path (23c). The first branch path (23b) has an outflow end connected to the first spraying nozzle (63). The second branch path (23c) has an outflow end connected to the second spraying nozzle (68).

The first on-off valve (24) is arranged on the first branch path (23b). The first on-off valve (24) is configured as an electromagnetic valve. The first on-off valve (24) is opened when air is sent to the first spraying unit (61). The second on-off valve (25) is arranged on the second branch path (23c). The second on-off valve (25) is configured as an electromagnetic valve. The second on-off valve (25) is opened when air is sent to the second spraying unit (66).

### (Filter)

The filters (26) are each arranged in the first branch path (23b) and the second branch path (23c). Specifically, one of the filters (26) is arranged between the first on-off valve (24) and the first spraying unit (61) in the first branch path (23b). The other one of the filters (26) is arranged between the second on-off valve (25) and the second spraying unit (66) in the second branch path (23c). The filter (26) is for collecting dust etc. contained in air sucked from the air pump (22).

### (Fragrance)

In this example, the first fragrance (C1) stored in the first tank (62) and the second fragrance (C2) stored in the second tank (67) are fragrances having an effect of promoting the state of the intestinal activity. In other words, the fragrances have an effect of enhancing the parasympathetic nerve of a human. Specifically, the first fragrance (C1) is a fragrance containing lavender as a main component. The second fragrance (C2) is a fragrance having a leaf aroma and containing leaf alcohol and leaf aldehyde as main components. The first fragrance and the second fragrance may contain fragrance components other than the above-described components.

### <Blower>

The blower (30) evenly distributes the fragrance released from the releaser (20) to the indoor space (S). The blower (30) is a fan that blows air. In this example, the blower (30) is arranged in the casing (21) of the releaser (20). The blower (30) blows air toward the indoor space (S).

### <Control Device>

As illustrated in FIG. 2, the regulator (3) of the intestinal activity regulation device (2) has the control device (40). The control device (40) includes a microcomputer and a memory device which are mounted on a control board. The memory device stores software for operating the microcomputer.

The control device (40) has a second communicator (41) and a determiner (42) as functional units. The second communicator (41) is connected to the communication terminal (50) via a wired or wireless communication line. The second communicator (41) is connected to the communication terminal (50) via short-range wireless communication such as the Bluetooth (registered trademark, the same applies hereinafter). The second communicator (41) includes a receiver that receives the intestinal sound data transmitted from the detection unit (10) via the communication terminal (50) and information for determining the release amount of the useful substance transmitted from the communication terminal (50). The second communicator (41) includes a transmitter that transmits the level of the intestinal activity to the communication terminal (50).

The determiner (42) determines the level of the intestinal activity based on the intestinal sound data received by the second communicator (41), and outputs the determined level of the intestinal activity. As the level of the intestinal activity, for example, a plurality of levels corresponding to the intestinal sound data is used. The more active the intestine is, the higher the level of the intestinal activity is.

The control device (40) controls the releaser (20) and the blower (30). Specifically, the control device (40) controls the air pump (22), the first on-off valve (24), and the second on-off valve (25) to cause the releaser (20) to perform regulation process. In the regulation process by the releaser (20), the useful substance is released from the releaser (20). The control device (40) controls the blower (30) to blow air in conjunction with the regulation process by the releaser (20).

In this example, the release amount of the useful substance is adjusted at the time interval of intermittent operation of the air pump (22). The release amount of the useful substance may be adjusted by adjusting the time interval of an opening-closing process by the on-off valves (24, 25), the discharge flow rate of the air pump (22), the opening degree of the air flow path (23), etc.

The control device (40) adjusts the release amount of the useful substance according to environment in the indoor space (S). In other words, the time interval of the intermittent operation of the air pump (22) is adjusted according to the environment in the indoor space (S). In this example, the size of a room and the type of building are used as the environment in the indoor space (S).

Specifically, as illustrated in FIG. 4, the release amount of the useful substance is changed depending on which size the room has among four tatami mats (six tatami mats or less), eight tatami mats (six to eight tatami mats), and 12 tatami mats (10 to 14 tatami mats). The release amount of the useful substance is changed depending on which type the building is among a wooden building, a prefabricated building, and other types of building. Here, the number of times of ventilation per hour varies depending on the type of building. Thus, the type of building affects adjustment of the release amount of the useful substance.

The control device (40) adjusts the release amount of the useful substance according to the type of useful substance. In other words, the time interval of the intermittent operation of the air pump (22) is adjusted according to the useful substance. In this example, the release amount of the useful substance is changed depending on which type of useful substance is used among the first fragrance (C1) stored in the first tank (62) or the second fragrance (C2) stored in the second tank (67).

The control device (40) adjusts the release amount of the useful substance according to the size of the room, the type of building, and the type of useful substance as described above. The size of the room, the type of building, and the type of useful substance to be released are set by the target person (T) via an operator (52) of the communication terminal (50) described later.

### <Communication Terminal>

As illustrated in FIG. 2, the intestinal activity regulation device (2) includes the communication terminal (50). The communication terminal (50) is a terminal operated by the target person (T). The communication terminal (50) of this example is a smartphone. The communication terminal (50) includes a microcomputer and a memory device. The memory device stores software for operating the microcomputer. The communication terminal (50) includes a third communicator (51), the operator (52), a display (53), and a storage (54) as functional units.

The communication terminal (50) functions as the third communicator (51), the operator (52), the display (53), and the storage (54) by executing a program recorded in the memory device. The program stored in the communication terminal (50) corresponds to the intestinal activity regulation program of the present disclosure. The program causes the communication terminal (50) as a computer to execute first processing, second processing, third processing, fourth processing, and fifth processing.

In the first processing, the state of the intestinal activity detected by the detector (11) is displayed on the display (53). In the second processing, the state of the intestinal activity detected by the detector (11) is transmitted to the regulator (3). In the third processing, the indication of the state of the intestinal activity on the display (53) is changed based on the regulation process by the regulator (3). In the fourth processing, information input via the operator (52) is transmitted to the regulator (3). In the fifth processing, the state of the intestinal activity detected by the detector (11) is stored in the storage (54).

### (Third Communicator)

The third communicator (51) includes a receiver that receives the intestinal sound data transmitted from the detection unit (10) and receives the level of the intestinal activity transmitted from the regulator (3). The third communicator (51) includes a transmitter that transmits the intestinal sound data to the regulator (3).

### (Operator)

The operator (52) is configured as a touch panel, for example. The target person (T) uses the operator (52) to operate application software stored in the communication terminal (50). With operation of this application software, it is possible to start or stop the intestinal activity regulation system (1), input a bedtime and a wake-up time, and input the information for determining the release amount of the useful substance.

Here, as the information for determining the release amount of the useful substance in this example, the target person (T) selects information for each of the size of the room, the type of building, and the type of useful substance as described above. Specifically, as illustrated in FIG. 4, the target person (T) selects one of three types of size as the type of room. The target person (T) selects one of three types of building as the type of building. The target person (T) selects one of two types of fragrance as the type of useful substance.

### (Display)

The display (53) is configured as a liquid crystal panel, for example. The display (53) displays the state of the intestinal activity detected by the detector (11). **In** this example, the display (53) displays the level of the intestinal activity as the state of the intestinal activity. The display (53) displays the state of the intestinal activity before the regulation process is performed and the state of the intestinal activity after the regulation process has been performed. The indication of the state of the intestinal activity before and after the regulation process on the display (53) will be described later.

### (Storage)

The storage (54) stores the state of the intestinal activity detected by the detector (11). In this example, the storage (54) stores the level of the intestinal activity as the state of the intestinal activity. The storage (54) includes a random access memory (RAM).

### -Indication of State of Intestinal Activity-

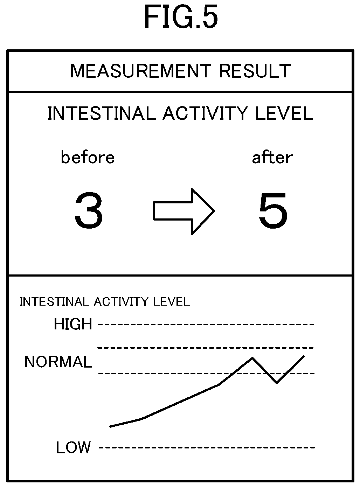

Next, the indication of the state of the intestinal activity before and after the regulation process on the display (53) will be described with reference to FIG. 5.

FIG. 5 is an image displayed on the display (53) of the communication terminal (50). At the top of the image, the state of the intestinal activity before and after the regulation process is displayed. Specifically, the state of the intestinal activity (in this example, the level of the intestinal activity) before the regulation process is performed is displayed on the left side at the top of the image.

The state of the intestinal activity (in this example, the level of the intestinal activity) after the regulation process has been performed is displayed on the right side at the top of the image. Here, the state of the intestinal activity after the regulation process has been performed in this example is the state of the intestinal activity detected immediately before operation of the intestinal activity regulation system (1) ends. With this configuration, the target person (T) can recognize the improvement status of the intestinal activity by using the intestinal activity regulation system (1).

At the bottom of the image, a change in the state of the intestinal activity (in this example, the level of the intestinal activity) measured in the past is displayed in a graph. The horizontal axis in the graph indicates a date and the vertical axis indicates the level of the intestinal activity. In the graph, the change in the level of the intestinal activity during any of the past one week, the past two weeks, and the past one month is displayed as a broken-line graph. With this configuration, the target person (T) can recognize the improvement status of the intestinal activity due to long-term use of the intestinal activity regulation system (1).

### -Operation of Intestinal Activity Regulation System-

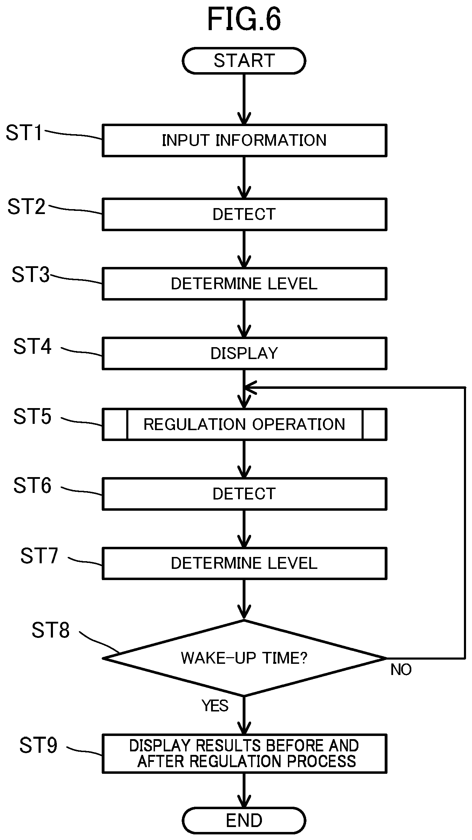

Operation of the intestinal activity regulation system (1) will be described with reference to FIG. 6. The intestinal activity regulation system (1) of this example operates from immediately before the target person (T) sleeps to immediately after the target person (T) has woken up.

As illustrated in FIG. 6, when the intestinal activity regulation system (1) starts operation, the target person (T) selects the size of the room, the type of building, and the type of useful substance to be released, and then inputs the wake-up time to the operator (52) of the communication terminal (50), in Step ST1. The information input to the operator (52) is transmitted to the third communicator (51). The third communicator (51) transmits, to the second communicator (41), the information input in the operator (52).

Next, in Step ST2, the detector (11) detects the state of the intestinal activity of the target person (T). Specifically, in Step ST2, the intestinal sound of the target person (T) is measured by the detector (11). The measured intestinal sound data is transmitted to the first communicator (12). The first communicator (12) transmits the measured intestinal sound data to the second communicator (41) via the third communicator (51).

Next, in Step ST3, the level of the intestinal activity is determined based on the state of the intestinal activity detected by the detector (11). Specifically, in Step ST3, the determiner (42) of the control device (40) determines the level of the intestinal activity based on the intestinal sound data received by the second communicator (41), and outputs the determined level of the intestinal activity. The output level of the intestinal activity is transmitted to the third communicator (51) via the second communicator (41). The level of the intestinal activity received by the third communicator (51) is stored in the storage (54).

Next, in Step ST4, the state of the intestinal activity of the target person (T) is displayed on the display (53). Specifically, in Step ST4, the level of the intestinal activity of the target person (T) received by the third communicator (51) is transmitted to the display (53). The display (53) having received the level of the intestinal activity displays the level of the intestinal activity of the target person (T).

Next, in Step ST5, the control device (40) performs the regulation operation. The control device (40) controls the releaser (20) and the blower (30) in the regulation operation. The regulation operation will be described later.

Next, in Step ST6, the detector (11) detects the state of the intestinal activity of the target person (T). Specifically, in Step ST6, the intestinal sound of the target person (T) is measured by the detector (11). The measured intestinal sound data is transmitted to the first communicator (12). The first communicator (12) transmits the measured intestinal sound data to the second communicator (41) via the third communicator (51).

Next, in Step ST7, the level of the intestinal activity is determined based on the state of the intestinal activity detected by the detector (11). Specifically, in Step ST7, the determiner (42) of the control device (40) determines the level of the intestinal activity based on the intestinal sound data received by the second communicator (41), and outputs the determined level of the intestinal activity. The output level of the intestinal activity is transmitted to the third communicator (51) via the second communicator (41). The level of the intestinal activity received by the third communicator (51) is stored in the storage (54).

Next, in Step ST8, the control device (40) determines whether or not the current time has passed the pre-input wake-up time. If the current time has passed the pre-input wake-up time (YES in Step ST8), the processing proceeds to Step ST9. If the current time does not pass the pre-input wake-up time (NO in Step ST8), the processing proceeds to Step ST5.

In Step ST9, the display (53) displays the state of the intestinal activity before the regulation process is performed and the state of the intestinal activity after the regulation process has been performed. Specifically, in Step ST9, the display (53) displays the level of the intestinal activity before the regulation process is performed, the latest level of the intestinal activity after the regulation process has been performed, and the change in the level of the intestinal activity measured in the past.

As described above, when operation of the intestinal activity regulation system (1) starts, the display (53) displays the state of the intestinal activity of the target person (T) before the regulation process is performed; and when operation of the intestinal activity regulation system (1) ends, the display (53) displays the state of the intestinal activity of the target person after the regulation process has been performed. In other words, the indication of the state of the intestinal activity on the display (53) is changed based on the regulation process by the regulator (3).

### <Regulation Operation>

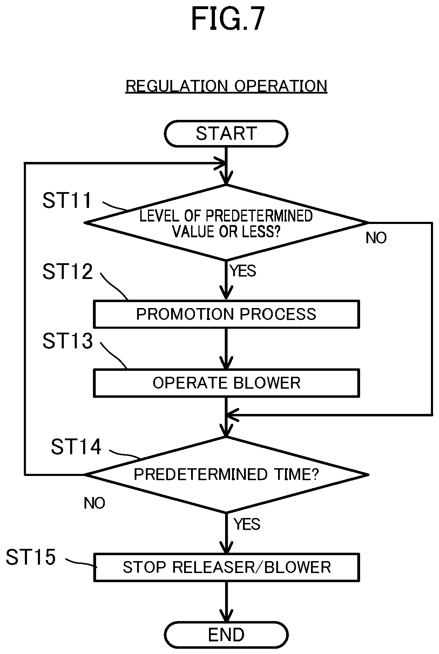

The regulation operation in Step ST5 of FIG. 6 will be described with reference to FIG. 7.

In Step ST11, the control device (40) determines whether or not the level of the intestinal activity is a predetermined value or less. If the level of the intestinal activity is the predetermined value or less (YES in Step ST11), the processing proceeds to Step ST12. If the level of the intestinal activity is greater than the predetermined value (NO in Step ST11), the processing proceeds to Step ST14.

In Step ST12, the control device (40) causes the releaser (20) to perform the regulation process based on the information input via the operator (52). In this example, the releaser (20) performs, as the regulation process, a promotion process of promoting the intestinal activity. In the promotion process, the releaser (20) releases the useful substance promoting the intestinal activity. A specific process by the releaser (20) will be described later.

Next, in Step ST13, the control device (40) operates the blower (30) in conjunction with the promotion process by the releaser (20). With this configuration, unevenness in the concentration of the useful substance released from the releaser (20) into the indoor space (S) is reduced.

Next, in Step ST14, the control device (40) determines whether or not a predetermined time has elapsed. If the predetermined time has elapsed (YES in Step ST14), the processing proceeds to Step ST15. If the predetermined time has not elapsed (NO in Step ST14), the processing proceeds to Step ST11.

In Step ST15, the control device (40) stops the processes by the releaser (20) and the blower (30).

### <Process by Releaser>

The releaser (20) performs a first process and a second process. The first process is a process of releasing the first fragrance (C1) into the indoor space (S). The second process is a process of releasing the second fragrance (C2) into the indoor space (S). The control device (40) switches between the first process and the second process based on the information input via the operator (52).

### (First Process)

In the first process, the air pump (22) is turned on, the first on-off valve (24) is opened, and the second on-off valve (25) is closed. Air delivered by the air pump (22) passes through the main flow path (23a), and flows into the first branch path (23b) via the first on-off valve (24). The air having passed through the first branch path (23b) flows into the first spraying nozzle (63). In the first spraying nozzle (63), the first fragrance (C1) is atomized by an airflow of the air. The atomized first fragrance (C1) is supplied to the indoor space (S) through the ejection port (65). Since the second on-off valve (25) is closed, the air delivered by the air pump (22) does not flow into the second spraying nozzle (68).

### (Second Process)

In the second process, the air pump (22) is turned on, the first on-off valve (24) is closed, and the second on-off valve (25) is opened. Air delivered by the air pump (22) passes through the main flow path (23a), and flows into the second branch path (23c) via the second on-off valve (25). The air having passed through the second branch path (23c) flows into the second spraying nozzle (68). In the second spraying nozzle (68), the second fragrance (C2) is atomized by such an airflow. The atomized second fragrance (C2) is supplied to the indoor space (S) through the ejection port (65). Since the first on-off valve (24) is closed, the air delivered by the air pump (22) does not flow into the first spraying nozzle (63).

As described above, the useful substance for promoting the intestinal activity of the target person (T) is released into the indoor space (S), and then the target person (T) inhales the useful substance with breathing. When the target person (T) inhales the useful substance, the parasympathetic nerve of the target person (T) is enhanced by the action of the useful substance, and the intestinal activity is promoted. As a result, the intestinal activity of the target person (T) is returned to a normal state, and a symptom (e.g., constipation) caused due to a decrease in the intestinal activity of the target person (T) is improved.

### -Features of Embodiment-

A feature (1) of this embodiment is that the intestinal activity regulation device (2) includes the regulator (3) that performs the regulation process of regulating the intestinal activity based on the state of the intestinal activity detected by the detector (11). According to this configuration, since the regulation process is performed by the regulator (3), the intestinal activity of the target person (T) can be regulated based on the state of the intestinal activity of the target person (T).

A feature (2) of this embodiment is that the regulator (3) performs, as the regulation process, the promotion process of promoting the intestinal activity. According to this configuration, the intestinal activity of the target person (T) is promoted.

A feature (3) of this embodiment is that the intestinal activity regulation device (2) includes the display (53). According to this configuration, since the state of the intestinal activity detected by the detector (11) is displayed on the display (53), it is possible to notify the target person (T) of the state of the intestinal activity.

A feature (4) of this embodiment is that the display (53) displays the state of the intestinal activity before the regulation process is performed and the state of the intestinal activity after the regulation process has been performed. According to this configuration, it is possible to notify the target person (T) of the change in the state of the intestinal activity by performing the regulation process.

A feature (5) of this embodiment is that the regulator (3) has the releaser (20). According to this configuration, the useful substance is released into the indoor space (S) where the target person (T) is present.

A feature (6) of this embodiment is that the regulator (3) causes the releaser (20) to release the useful substance for promoting the intestinal activity when the level of the intestinal activity is the predetermined value or less. According to this configuration, when the level of the intestinal activity of the target person (T) is low, the intestinal activity of the target person (T) can be promoted. For example, if the constipation symptom occurs when the intestinal activity of the target person (T) is weaker than that in the normal state, the target person (T) inhales the useful substance released from the releaser (20), so that the parasympathetic nerve of the target person (T) is enhanced and the intestinal activity is promoted accordingly. As a result, the constipation symptom of the target person (T) is alleviated.

A feature (7) of this embodiment is that the intestinal activity regulation device (2) includes the blower (30). According to this configuration, since the blower (30) blows air in conjunction with the regulation process, it is possible to reduce the unevenness in the concentration of the useful substance released from the regulator (3).

A feature (8) of this embodiment is that the regulator (3) adjusts the release amount of the useful substance according to the environment in the indoor space (S). According to this configuration, the release amount of the useful substance is adjusted according to the environment in the indoor space (S).

A feature (9) of this embodiment is that the regulator (3) adjusts the release amount of the useful substance according to the type of useful substance. According to this configuration, the release amount of the useful substance is adjusted according to the type of useful substance.

A feature (10) of this embodiment is that the intestinal activity regulation device (2) is operated while the target person (T) is sleeping. According to this configuration, since the intestinal activity is improved while the target person (T) is sleeping, it is easy to reactivate the intestine after the target person (T) has woken up (particularly after breakfast) and it is possible to promote normal defecation.

### -Variations of Embodiment-

### <First Variation>

In the intestinal activity regulation device (2) of this embodiment, an inhibition process may be performed as the regulation process in the regulation operation. In the inhibition process, the releaser (20) releases a useful substance that inhibits the intestinal activity. Specifically, the second tank (67) of the spraying unit (66) stores a second fragrance having an effect of suppressing the state of the intestinal activity.

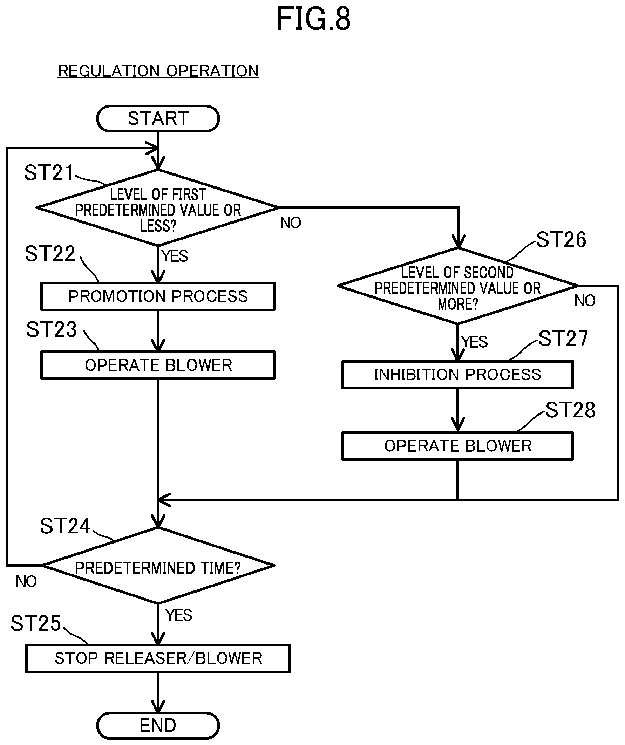

**In** addition, as illustrated in FIG. 8, in the intestinal activity regulation device (2) of this embodiment, the promotion process and the inhibition process may be performed as the regulation processes in the regulation operation. The control device (40) causes the releaser (20) to perform the promotion process or the inhibition process based on the level of the intestinal activity.

Specifically, in Step ST21, the control device (40) determines whether or not the level of the intestinal activity is a first predetermined value or less. If the level of the intestinal activity is the first predetermined value or less (YES in Step ST21), the processing proceeds to Step ST22. If the level of the intestinal activity is greater than the first predetermined value (NO in Step ST21), the processing proceeds to Step ST24.

In Step ST22, the control device (40) causes the releaser (20) to perform the promotion process based on the level of the intestinal activity. Specifically, the releaser (20) releases the first fragrance (C1) having the effect of promoting the state of the intestinal activity into the indoor space (S).

Next, in Step ST23, the control device (40) operates the blower (30) in conjunction with the promotion process by the releaser (20). With this configuration, the unevenness in the concentration of the useful substance released from the releaser (20) into the indoor space (S) is reduced.

Next, in Step ST24, the control device (40) determines whether or not a predetermined time has elapsed. If the predetermined time has elapsed (YES in Step ST24), the processing proceeds to Step ST25. If the predetermined time has not elapsed (NO in Step ST24), the processing proceeds to Step ST21.

In Step ST25, the control device (40) stops the processes by the releaser (20) and the blower (30).

In Step ST26, the control device (40) determines whether or not the level of the intestinal activity is a second predetermined value or more. If the level of the intestinal activity is the second predetermined value or more (YES in Step ST26), the processing proceeds to Step ST27. If the level of the intestinal activity is less than the second predetermined value (NO in Step ST26), the processing proceeds to Step ST24.

In Step ST27, the control device (40) causes the releaser (20) to perform the inhibition process based on the level of the intestinal activity. Specifically, the releaser (20) releases the second fragrance (C2) having an effect of suppressing the state of the intestinal activity into the indoor space (S).

Next, in Step ST28, the control device (40) operates the blower (30) in conjunction with the inhibition process by the releaser (20). With this configuration, the unevenness in the concentration of the useful substance released from the releaser (20) into the indoor space (S) is reduced. After the end of Step ST28, the processing proceeds to Step ST24.

If the level of the intestinal activity is the first predetermined value or less, the intestinal activity is in a low state, and therefore, symptoms such as constipation are likely to occur. In such a case, the intestinal activity of the target person (T) can be promoted and be returned to the normal state by release of the first fragrance from the releaser (20).

If the level of the intestinal activity is the second predetermined value or more, the intestinal activity is in an extremely high state, and therefore, symptoms such as diarrhea are likely to occur. In such a case, the intestinal activity of the target person (T) can be suppressed and be returned to the normal state by release of the second fragrance from the releaser (20).

If the level of the intestinal activity is greater than the first predetermined value and less than the second predetermined value, the intestinal activity of the target person (T) is in the normal state, and therefore, the regulation process by the releaser (20) is not performed.

### <Second Variation>

In the intestinal activity regulation device (2) of this embodiment, the target person (T) may manually adjust the intensity of the aroma released from the releaser (20). Specifically, the target person (T) uses the operator (52) to operate the application software recorded in the communication terminal (50), in order to change the intensity of the aroma of the useful substance to be released.

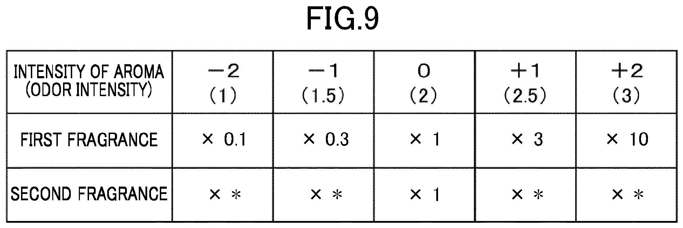

In this example, as illustrated in FIG. 9, the intensity of the aroma is divided into five levels of intensity. The target person (T) selects one of the five levels of intensity. Here, the intensity of the aroma automatically set by the control device (40) of the intestinal activity regulation device (2) is used as a reference, and is taken as zero. The reference intensity of the aroma is, for example, an odor intensity of 2. The odor intensity described here is an intensity in the six grades odor intensity measurement method. The odor intensity of 2 in the six grades odor intensity measurement method is a weak odor to such an extent that the type of odor can be recognized.

If the intensity of the aroma is increased by one level from the reference aroma via the operator (52), an aroma having an odor intensity of about 2.5 is released from the releaser (20). If the intensity of the aroma is increased by two levels, an aroma having an odor intensity of about 3 is released from the releaser (20). The odor intensity of 3 in the six grades odor intensity measurement method is an odor which can be easily sensed.

On the other hand, if the intensity of the aroma is decreased by one level from the reference aroma via the operator (52), an aroma having an odor intensity of about 1.5 is released from the releaser (20). If the intensity of the aroma is decreased by two levels, an aroma having an odor intensity of about 1 is released from the releaser (20). The odor intensity of 1 in the six grades odor intensity measurement method is an odor which can be barely sensed.

The intensity of the aroma is adjusted by adjusting the release amount of the useful substance. Here, if the release amount of the useful substance is adjusted by the time interval of the intermittent operation of the air pump (22), the intensity of the aroma is adjusted by changing a time for which the air pump (22) is ON.

Specifically, for example, if the intensity of the aroma is to be increased by one level from the reference aroma, the time for which the air pump (22) is ON is multiplied by three. If the intensity of the aroma is increased by two levels, the time for which the air pump (22) is ON is multiplied by 10.

On the other hand, for example, if the intensity of the aroma is to be decreased by one level from the reference aroma, the time for which the air pump (22) is ON is multiplied by 0.3. If the intensity of the aroma is decreased by two levels, the time for which the air pump (22) is ON is multiplied by 0.1.

Thus, the intensity of the aroma of the useful substance released from the intestinal activity regulation device (2) can be adjusted according to the preference of the target person (T). The odor intensity and the time for which the air pump (22) is ON as described here are merely examples. The multiplying factor of the time for which the air pump (22) is ON may vary depending on the type of fragrance as the useful substance to be released.

### <Third Variation>

The intestinal activity regulation device (2) of this embodiment may have a heater instead of the releaser (20). The heater is a heating device that generates heat to about 40°C which is slightly higher than a body temperature. The temperature of the heating device increases, for example, when a heat generation element or a Peltier element generates heat. The heater is small and portable. The heater is attached in close contact with the abdomen (around the intestines) of the target person (T). When the heater generates heat, the abdomen of the target person (T) is warmed.

The heater is controlled by the control device (40). Specifically, in the regulation operation of operation of the intestinal activity regulation system (1), the control device (40) causes the heater to perform the promotion process. In the promotion process, the heater generates heat to promote the intestinal activity. In the promotion process, the control device (40) changes the heating temperature of the heater according to the level of the intestinal activity.

As described above, since the intestinal activity regulation device (2) has the heater, the intestinal activity can be promoted by directly warming the abdomen of the target person (T). The heater may be configured integrally with the detector (11) or be configured separately therefrom.

### <Fourth Variation>

In the intestinal activity regulation device (2) of this embodiment, the releaser (20) may include a second display. The second display is configured as, for example, a liquid crystal panel. The second display in the releaser (20) displays a regulation method for regulating the intestinal activity based on the state of the intestinal activity detected by the detector (11). Examples of the regulation method displayed on the second display include an exercise for promoting the intestinal activity, a lifestyle improvement method for promoting the intestinal activity, and a regulation method using a product in accordance with the state of the intestinal activity. When the regulation method using the product in accordance with the state of the intestinal activity is displayed, detailed information on the product to be used may be displayed. The regulation method for regulating the intestinal activity may be displayed on the display (53) of the communication terminal (50). The second display may display the level of the intestinal activity output from the determiner (42) of the control device (40).

### <Fifth Variation>

**In** the intestinal activity regulation device (2) of this embodiment, the regulator (3) may perform the regulation process so as to act on the autonomic nerve of the target person (T).

Specifically, if the regulator (3) has the releaser (20), the releaser (20) releases a useful substance acting on the autonomic nerve of the target person (T). Specifically, the first fragrance (C1) and the second fragrance (C2) of the above-described embodiment contain the useful substances that act on the autonomic nerve of the target person (T) to promote the state of the intestinal activity of the target person (T).

**In** this case, when the target person (T) inhales the first fragrance (C1) or the second fragrance (C2) released from the releaser (20), the odorant of the fragrance is sensed by the olfactory receptor in the nasal cavity of the target person (T). Then, the information obtained by such sensing is transmitted to the center of the autonomic nerve in the brain of the target person (T), whereby the sympathetic nerve is suppressed and, at the same time, the parasympathetic nerve becomes dominant and is enhanced. As a result, the intestinal activity of the target person (T) can be promoted.

If the regulator (3) uses the first fragrance (C1) or the second fragrance (C2) to perform the inhibition process, the first fragrance (C1) or the second fragrance (C2) may contain a useful substance that acts on the autonomic nerve of the target person (T) to suppress the state of the intestinal activity of the target person (T).

If the regulator (3) includes the heater instead of the releaser (20) as in the third variation, the heater warms the abdomen of the target person (T) to act on the autonomic nerve of the target person (T). Specifically, when the abdomen of the target person (T) is warmed by heat generation of the heater, heat stimuli are given to the abdomen, and a skin temperature increases. When the skin temperature of the target person (T) increases, the thermosensory receptors are stimulated, and excitation due to such stimulation acts on the center of the autonomic nerve in the brain. Accordingly, the sympathetic nerve is suppressed, and at the same time, the parasympathetic nerve becomes dominant and is enhanced. Thus, the intestinal activity of the target person (T) is promoted.

### <Sixth Variation>

In the intestinal activity regulation device (2) of this embodiment, when the regulator (3) has the heater instead of the releaser (20) as in the third variation, the heating device serving as the heater may uses a semiconductor having a temperature self-regulation function. The heating device may be, for example, a PTC heater or a heater cable configured such that semiconductor heating elements form a parallel circuit. Even if such a heating device is used, the heater directly warms the abdomen of the target person (T) to give heat stimuli to the abdomen, thereby acting on the autonomic nerve of the target person (T). Accordingly, the intestinal activity of the target person (T) is promoted.

### <Seventh Variation>

In the intestinal activity regulation device (2) of this embodiment, when the regulator (3) has the heater instead of the releaser (20) as in the third variation, the heater does not necessarily closely contact the abdomen of the target person (T). In other words, the heater may apply heat from a location apart from the abdomen of the target person (T). Specifically, the heating device serving as the heater is, for example, a halogen heater, a carbon heater, a graphite heater, or a sheathed heater. If such a heater is used, the heater is directed toward the abdomen of the target person (T) to warm the periphery of the abdomen of the target person (T). Accordingly, heat stimuli are given to the abdomen of the target person (T) and act on the autonomic nerve of the target person (T), thereby promoting the intestinal activity of the target person (T).

### <Eighth Variation>

In the intestinal activity regulation device (2) of this embodiment, the regulator (3) may have a vibrator instead of the releaser (20). The vibrator is a vibration device that generates vibration. The vibration device is, for example, an ultrasonic motor, a vibration motor, a crystal vibrator, or an oscillator. The vibration device vibrates in small increments. The vibrator is small and portable. The vibrator is attached in close contact with the abdomen (around the intestines) of the target person (T). When the vibrator vibrates, stroking stimuli are given to the abdomen of the target person (T). In this manner, direct stimuli are given to the abdomen of the target person (T) by the vibrator.

The vibrator is controlled by the control device (40). Specifically, in the regulation operation of the intestinal activity regulation system (1), the control device (40) causes the vibrator to perform the promotion process. In the promotion process, the vibrator vibrates to promote the intestinal activity of the target person (T). In the promotion process, the control device (40) changes the frequency of the vibrator according to the level of the intestinal activity.

As described above, in this variation, since the intestinal activity regulation device (2) has the vibrator, direct stroking stimuli are given to the abdomen of the target person (T). Accordingly, the direct stimuli generated by vibration act on the autonomic nerve of the target person (T), thereby promoting the intestinal activity of the target person (T). The vibrator may be configured integrally with the detector (11) or be configured separately therefrom.

### <Ninth Variation>

In the intestinal activity regulation device (2) of this embodiment, the regulator (3) may have an electric stimulator instead of the releaser (20). The electric stimulator is an electric device that applies weak electricity to a human body. The electric stimulator is small and portable. The electric stimulator is attached in close contact with the abdomen (around the intestines) of the target person (T). When the weak electricity is applied to the abdomen of the target person (T) by the electric stimulator, electric stimuli are given to the abdomen of the target person (T). In this manner, direct stimuli are given to the abdomen of the target person (T) by the electric stimulator.

The electric stimulator is controlled by the control device (40). Specifically, in the regulation operation of the intestinal activity regulation system (1), the control device (40) causes the electric stimulator to perform the promotion process. In the promotion process, the electric stimulator applies the electricity to the target person (T) to promote the intestinal activity of the target person (T).

As described above, in this variation, since the intestinal activity regulation device (2) has the electric stimulator, direct electric stimuli are given to the abdomen of the target person (T). Accordingly, the direct stimuli generated by the electricity act on the autonomic nerve of the target person (T), thereby promoting the intestinal activity of the target person (T). The electric stimulator may be configured integrally with the detector (11) or be configured separately therefrom.

### <Tenth Variation>

In the intestinal activity regulation device (2) of this embodiment, the regulator (3) may have a press instead of the releaser (20). The press is small and portable. The press is, for example, an airbag. The airbag is wrapped around the abdomen of the target person (T) with a belt which is attached to the airbag. The airbag is attached in close contact with the abdomen (around the intestines) of the target person (T). When the airbag inflates, pressing stimuli are given to the abdomen of the target person (T). In this manner, direct stimuli are given to the abdomen of the target person (T) by the press.

The press is controlled by the control device (40). Specifically, in the regulation operation of the intestinal activity regulation system (1), the control device (40) causes the press to perform the promotion process. In the promotion process, the airbag serving as the press inflates to promote the intestinal activity of the target person (T).

As described above, in this variation, since the intestinal activity regulation device (2) has the press, direct pressing stimuli are given to the abdomen of the target person (T). Accordingly, the pressing stimuli generated by vibration act on the autonomic nerve of the target person (T), thereby promoting the intestinal activity of the target person (T). The press may be configured integrally with the detector (11) or be configured separately therefrom.

### <Eleventh Variation>

In the intestinal activity regulation device (2) of this embodiment, the regulator (3) may have a sound generator instead of the releaser (20). The sound generator generates sound for regulating the intestinal activity. The sound generator is configured as, for example, a music reproduction device such as a digital audio player. The sound generator may have the speaker function of the communication terminal (50). The sound generator may generate sound via a speaker or earphones.

The sound generator is controlled by the control device (40). Specifically, in the regulation operation of the intestinal activity regulation system (1), the control device (40) causes the sound generator to perform the promotion process or the inhibition process. In the promotion process, the sound generator plays a music to promote the intestinal activity of the target person (T). In the inhibition process, the sound generator plays a music to inhibit the intestinal activity of the target person (T).

Specifically, in the promotion process, the music played by the sound generator is a healing music or a sound containing a 1/f fluctuation (e.g., natural sound, wave sound, small bird sound, and babbling stream sound). Here, the 1/f fluctuation is a fluctuation in which a spectral density is inversely proportional to a frequency f. In the inhibition process, the music played by the sound generator is an avant-garde music or a hard lock music.

As described above, in this variation, since the intestinal activity regulation device (2) has the sound generator, audio stimuli are given to the target person (T) by the music played by the sound generator. When the sound generator performs the promotion process, the audio stimuli by the music act on the autonomic nerve of the target person (T), and accordingly, the parasympathetic nerve becomes dominant. As a result, the intestinal activity of the target person (T) can be promoted. On the other hand, when the sound generator performs the inhibition process, the audio stimuli by the music act on the autonomic nerve of the target person (T), and accordingly, the sympathetic nerve becomes dominant. As a result, the intestinal activity of the target person (T) can be inhibited.

### <Twelfth Variation>

In the intestinal activity regulation device (2) of this embodiment, the regulator (3) may have a video presenter instead of the releaser (20). The video presenter presents a video for regulating the intestinal activity. The video presenter is configured as, for example, a liquid crystal panel, a touch panel, or a projector. The video presenter may be configured as the display (53) of the communication terminal (50). The video presented by the video presenter may be a still image or a moving image. The video presenter is arranged at a position at which the target person (T) can visually check the video presenter.

The video presenter is controlled by the control device (40). Specifically, in the regulation operation of the intestinal activity regulation system (1), the control device (40) causes the video presenter to perform the promotion process. In the promotion process, the video presenter present the video to promote the intestinal activity of the target person (T). Specifically, the video presented by the video presenter in the promotion process is, for example, a moving image causing spontaneous laughter (stand-up comedy moving image, comedy movie, etc.), an image of a natural scene including forests, flowers, etc., or an image of an ornamental plant.

If the moving image causing the spontaneous laughter is presented, laughter stimuli cause spontaneous laughter to make the target person (T) express the emotion, and therefore, the activity of the sympathetic nerve is enhanced. Thereafter, as the sympathetic nerve is depressed, the parasympathetic nerve is enhanced, and accordingly it becomes dominant. If the image of the natural scene or the ornamental plant is presented, visual stimuli by the image act on the autonomic nerve of the target person (T), and accordingly, the parasympathetic nerve becomes dominant.

As described above, in this variation, since the intestinal activity regulation device (2) has the video presenter, the visual stimuli are given to the target person (T) by the video presented by the video presenter. When the visual stimuli act on the autonomic nerve of the target person (T), the parasympathetic nerve becomes dominant. As a result, the intestinal activity of the target person (T) can be promoted. The video presenter may be configured integrally with the detector (11) or be configured separately therefrom.

### <Thirteenth Variation>

In the intestinal activity regulation device (2) of this embodiment, the regulator (3) may have an information presenter instead of the releaser (20). The information presenter presents information for regulating the intestinal activity. The information presenter is configured as, for example, a liquid crystal panel, a touch panel, or a projector. The information presenter may be configured as the display (53) of the communication terminal (50). The information presenter is arranged at a position at which the target person (T) can visually check the information presenter.

The information presenter is controlled by the control device (40). Specifically, in the regulation operation of the intestinal activity regulation system (1), the control device (40) causes the information presenter to perform the promotion process. In the promotion process, the information presenter presents information on action to promote the intestinal activity of the target person (T). Specifically, the information presented by the information presenter in the promotion process is, for example, the following (a1) to (a4).

(a1) Drink herbal tea (e.g. chamomile tea), plain hot water, or beverage at about 60°C;
(a2) Ingest supplements or drugs that promote the intestinal activity;
(a3) How to massage, press therapeutic points, or breathe in abdominally to promote the intestinal activity; and
(a4) Postural change from the supine position to the right lateral decubitus position.

As described above, in this variation, since the intestinal activity regulation device (2) has the information presenter, the target person (T) makes the presented action based on the information presented from the information presenter, so that the intestinal activity of the target person (T) can be promoted. The information presenter may be configured integrally with the detector (11) or be configured separately therefrom.

### <Fourteenth Variation>

In the intestinal activity regulation device (2) of this embodiment, the regulator (3) may have, as functional units, multiple ones of the releaser (20), the heater, the vibrator, the electric stimulator, the press, the sound generator, the video presenter, and the information presenter. Also in this case, it is possible to obtain effects similar to those of the above-described embodiment.

### <<Other Embodiments>>

The foregoing embodiment may be modified as follows.

In the intestinal activity regulation system (1) of the above-described embodiment, the detector (11) may be an ultrasonic sensor instead of the microphone. In this case, the peristaltic motion of the intestine is measured by ultrasonic sound.

In the intestinal activity regulation device (2) of the above-described embodiment, the useful substance to be released may be a substance other than the fragrance as long as the useful substance has the effect of promoting or inhibiting the intestinal activity. The useful substance to be released may be, for example, a pharmacologically active substance.

The intestinal activity regulation device (2) of the above-described embodiment does not necessarily include the blower (30). The blower (30) may be provided separately from the releaser (20), and be, for example, an air conditioner.

In the intestinal activity regulation system (1) of the above-described embodiment, the determiner (42) may be provided in the communication terminal (50) or a server device.

In the intestinal activity regulation device (2) of the above-described embodiment, the control device (40) may be provided in the server device or the communication terminal (50).

In the intestinal activity regulation system (1) of the above-described embodiment, the first communicator (12) may transmit the intestinal sound data measured by the detector (11) directly to the second communicator (41) without the third communicator (51).

In the intestinal activity regulation system (1) of the above-described embodiment, operation may be started at the bedtime that by the target person (T) inputs to the operator (52) and sets in advance. In this case, the bedtime is input to the operator (52) of the communication terminal (50) in advance.

In the intestinal activity regulation system (1) of the above-described embodiment, operation may be stopped after a lapse of a predetermined time since the start of the operation. The predetermined time described here may be a time initially set in advance in the intestinal activity regulation device (2) or a time input to the operator (52) by the target person (T).

In the intestinal activity regulation system (1) of the above-described embodiment, operation may be started or stopped by operating a switch provided in the detector (11), a switch provided in the intestinal activity regulation device (2), or application software stored in the communication terminal (50).

The intestinal activity regulation system (1) of the above-described embodiment may be operated in a time period (e.g., during reading or desk work) in which the target person (T) can efficiently inhale the useful substance in a resting state.

The ordinal numbers such as "first," "second," and "third" described above are used to distinguish the terms to which these expressions are given, and do not limit the number and order of the terms.

### INDUSTRIAL APPLICABILITY

As described above, the present disclosure is useful for the intestinal activity regulation device, the intestinal activity regulation system, and the intestinal activity regulation program.

### DESCRIPTION OF REFERENCE CHARACTERS

- 1: Intestinal Activity Regulation System
- 2: Intestinal Activity Regulation Device
- 3: Regulator
- 11: Detector
- 20: Releaser
- 30: Blower
- 53: Display
- T: Target Person
- S: Indoor Space (Space)

## Claims

1. An intestinal activity regulation device for improving intestinal activity of a target person (T), comprising:
a regulator (3) that performs a regulation process of regulating the intestinal activity based on a state of the intestinal activity detected by a detector (11) that detects the state of the intestinal activity of the target person (T),
the regulator (3) having a releaser (20) that releases a first fragrance (C1) or a second fragrance (C2), each of which is a useful substance for promoting the intestinal activity, into a space (S) where the target person (T) is present,
the first fragrance (C1) being a fragrance containing lavender as a main component,
the second fragrance (C2) being a fragrance containing leaf alcohol and leaf aldehyde as main components.

2. The intestinal activity regulation device of claim 1, wherein
the regulator (3) performs the regulation process so as to act on an autonomic nerve of the target person (T).

3. The intestinal activity regulation device of claim 1 or 2, wherein
the regulator (3) performs, as the regulation process, a promotion process of promoting the intestinal activity.

4. The intestinal activity regulation device of any one of claims 1 to 3, wherein the regulator (3) performs, as the regulation process, an inhibition process of inhibiting the intestinal activity.

5. The intestinal activity regulation device of any one of claims 1 to 4, further comprising:
a display (53) that displays the state of the intestinal activity detected by the detector (11).

6. The intestinal activity regulation device of claim 5, wherein
the display (53) displays the state of the intestinal activity before the regulation process and the state of the intestinal activity after the regulation process.

7. The intestinal activity regulation device of claim 1, wherein
the regulator (3) causes the releaser (20) to release the useful substance for promoting the intestinal activity when a level of the intestinal activity detected by the detector (11) is a predetermined value or less.

8. The intestinal activity regulation device of claim 1 or 7, wherein
the regulator (3) further has a blower (30) that blows air in conjunction with the regulation process.

9. The intestinal activity regulation device of claim 1, 7, or 8, wherein
the regulator (3) adjusts a release amount of the useful substance according to environment in the space (S).

10. The intestinal activity regulation device of any one of claims 1 and 7 to 9, wherein
the releaser (20) has a plurality of useful substances of different types, and
the regulator (3) adjusts the release amount of the useful substance according to the type of useful substance.

11. An intestinal activity regulation system comprising:
a detector (11) that detects the state of intestinal activity of a target person (T), and
the intestinal activity regulation device (2) of any one of claims 1 to 10.

## Patentansprüche

1. Vorrichtung zur Regulierung der Darmaktivität zur Verbesserung der Darmaktivität einer Zielperson (T), umfassend:
eine Regulierungseinrichtung (3), die einen Regulierungsprozess zur Regulierung der Darmaktivität basierend auf einem Zustand der Darmaktivität durchführt, der von einem Detektor (11) erfasst wird, der den Zustand der Darmaktivität der Zielperson (T) erfasst,
wobei die Regulierungseinrichtung (3) eine Freisetzeinrichtung (20) aufweist, die einen ersten Duftstoff (C1) oder einen zweiten Duftstoff (C2), von denen jeder eine nützliche Substanz zur Förderung der Darmaktivität ist, in einen Raum (S) freisetzt, in dem die Zielperson (T) anwesend ist,
wobei der erste Duftstoff (C1) ein Duftstoff ist, der Lavendel als eine Hauptkomponente enthält,
wobei der zweite Duftstoff (C2) ein Duftstoff ist, der Blattalkohol und Blattaldehyd als Hauptkomponenten enthält.

2. Vorrichtung zur Regulierung der Darmaktivität nach Anspruch 1, wobei
die Regulierungseinrichtung (3) den Regulierungsprozess durchführt, um auf einen autonomen Nerv der Zielperson (T) einzuwirken.

3. Vorrichtung zur Regulierung der Darmaktivität nach Anspruch 1 oder 2, wobei
die Regulierungseinrichtung (3) als den Regulierungsprozess einen Förderungsprozess zur Förderung der Darmaktivität durchführt.

4. Vorrichtung zur Regulierung der Darmaktivität nach einem der Ansprüche 1 bis 3, wobei
die Regulierungseinrichtung (3) als den Regulierungsprozess einen Hemmungsprozess zur Hemmung der Darmaktivität durchführt.

5. Vorrichtung zur Regulierung der Darmaktivität nach einem der Ansprüche 1 bis 4, ferner umfassend:
eine Anzeige (53), die den Zustand der Darmaktivität anzeigt, der von dem Detektor (11) erfasst wird.

6. Vorrichtung zur Regulierung der Darmaktivität nach Anspruch 5, wobei
die Anzeige (53) den Zustand der Darmaktivität vor dem Regulierungsprozess und den Zustand der Darmaktivität nach dem Regulierungsprozess anzeigt.

7. Vorrichtung zur Regulierung der Darmaktivität nach Anspruch 1, wobei
die Regulierungseinrichtung (3) die Freisetzeinrichtung (20) veranlasst, die nützliche Substanz zur Förderung der Darmaktivität freizusetzen, wenn ein Pegel der Darmaktivität, der von dem Detektor (11) erfasst wird, ein vorbestimmter Wert oder weniger ist.

8. Vorrichtung zur Regulierung der Darmaktivität nach Anspruch 1 oder 7, wobei
die Regulierungseinrichtung (3) ferner ein Gebläse (30) aufweist, das Luft in Verbindung mit dem Regulierungsprozess bläst.

9. Vorrichtung zur Regulierung der Darmaktivität nach Anspruch 1, 7 oder 8, wobei
die Regulierungseinrichtung (3) eine Freisetzungsmenge der nützlichen Substanz gemäß der Umgebung in dem Raum (S) einstellt.

10. Vorrichtung zur Regulierung der Darmaktivität nach einem der Ansprüche 1 und 7 bis 9, wobei
die Freisetzeinrichtung (20) eine Vielzahl von nützlichen Substanzen unterschiedlicher Arten aufweist, und
die Regulierungseinrichtung (3) die Freisetzungsmenge der nützlichen Substanz gemäß der Art der nützlichen Substanz einstellt.

11. System zur Regulierung der Darmaktivität, umfassend:
einen Detektor (11), der den Zustand der Darmaktivität einer Zielperson (T) erfasst, und
die Vorrichtung zur Regulierung der Darmaktivität (2) nach einem der Ansprüche 1 bis 10.

## Revendications

1. Dispositif de régulation de l'activité intestinale pour améliorer l'activité intestinale d'une personne cible (T), comprenant :
un régulateur (3) effectuant un processus de régulation pour la régulation de l'activité intestinale, en fonction d'un état de l'activité intestinale détecté par un détecteur (11) détectant l'état de l'activité intestinale de la personne cible (T),
le régulateur (3) possédant un déclencheur (20) libérant un premier parfum (C1) ou un deuxième parfum (C2), chacun desquels étant une substance utile pour la promotion de l'activité intestinale, dans un espace (S) où est présente la personne cible (T),
le premier parfum (C1) étant un parfum dont le principal composant est de la lavande,
le deuxième parfum (C2) étant un parfum dont les principaux composants sont un alcool extrait de feuilles et de l'aldéhyde de feuilles.

2. Dispositif de régulation de l'activité intestinale selon la revendication 1,
le régulateur (3) effectuant le processus de régulation de façon à intervenir sur un nerf autonome de la personne cible (T).

3. Dispositif de régulation de l'activité intestinale selon la revendication 1 ou 2,
le régulateur (3) effectuant, en tant que processus de régulation, un processus de promotion pour la promotion de l'activité intestinale.

4. Dispositif de régulation de l'activité intestinale selon une quelconque des revendications 1 à 3,
le régulateur (3) effectuant, en tant que processus de régulation, un processus d'inhibition pour l'inhibition de l'activité intestinale.

5. Dispositif de régulation de l'activité intestinale selon une quelconque des revendications 1 à 4, comprenant en outre :
un dispositif de visualisation (53) affichant l'état de l'activité intestinale détectée par le détecteur (11).

6. Dispositif de régulation de l'activité intestinale selon la revendication 5,
le dispositif de visualisation (53) affichant l'état de l'activité intestinale avant le processus de régulation et l'état de l'activité intestinale après le processus de régulation.

7. Dispositif de régulation de l'activité intestinale selon la revendication 1,
le régulateur (3) amenant le déclencheur (20) à libérer la substance utile pour promouvoir l'activité intestinale lorsqu'un niveau de l'activité intestinale détectée par le détecteur (11) est égal ou inférieur à une valeur prédéterminée.

8. Dispositif de régulation de l'activité intestinale selon la revendication 1 ou 7,
le régulateur (3) possédant en outre une soufflante (30) soufflant de l'air conjointement avec le processus de régulation.

9. Dispositif de régulation de l'activité intestinale selon la revendication 1, 7 ou 8,
le régulateur (3) ajustant une quantité libérée de la substance utile en fonction de l'environnement dans l'espace (S).

10. Dispositif de régulation de l'activité intestinale selon une quelconque des revendications 1 et 7 à 9,
le déclencheur (20) possédant une pluralité de substances utiles de différents types, et
le régulateur (3) ajustant la quantité libérée de la substance utile en fonction du type de substance utile.

11. Système de régulation de l'activité intestinale comprenant :
un détecteur (11) détectant l'état de l'activité intestinale d'une personne cible (T), et le dispositif de régulation de l'activité intestinale (2) selon une quelconque des revendications 1 à 10.
